(19) Europäisches Patentamt
European Patent Office
Office européen des brevets

(11) EP 1 209 141 B1

(12) **EUROPÄISCHE PATENTSCHRIFT**

(45) Veröffentlichungstag und Bekanntmachung des Hinweises auf die Patenterteilung:
**01.06.2005 Patentblatt 2005/22**

(51) Int Cl.[7]: **C07C 29/70**, C07C 31/30

(21) Anmeldenummer: **01127605.2**

(22) Anmeldetag: **20.11.2001**

# (54) Verfahren zur Herstellung von Magnesiumalkoholaten

Process for the preparation of magnesium alcoholates

Procédé de préparation d'alcoolates de magnésium

(84) Benannte Vertragsstaaten:
**AT BE CH CY DE DK ES FI FR GB GR IE IT LI LU MC NL PT SE TR**

(30) Priorität: **23.11.2000 DE 10058286**

(43) Veröffentlichungstag der Anmeldung:
**29.05.2002 Patentblatt 2002/22**

(73) Patentinhaber: **Chemetall GmbH
60487 Frankfurt (DE)**

(72) Erfinder:
- **Wietelmann, Ulrich, Dr.
61381 Friedrichsdorf (DE)**
- **Hauk, Dieter
61169 Friedberg (DE)**
- **Rittmeyer, Peter, Dr.
65843 Sulzbach/Taunus (DE)**

(74) Vertreter: **Uppena, Franz, Dr.
c/o Chemetall GmbH
Patente, Marken & Lizenzen
Trakehner Strasse 3
60487 Frankfurt am Main (DE)**

(56) Entgegenhaltungen:
**WO-A-99/58482**
**DE-A- 2 261 386**
**US-A- 3 388 179**

- **THOMS H ET AL: "The thermal decomposition of magnesium alcoholates to magnesia (MgO): studies by IR and thermal analysis" SOLID STATE IONICS, NORTH HOLLAND PUB. COMPANY. AMSTERDAM, NL, Bd. 101-103, Nr. 2001, 1. November 1997 (1997-11-01), Seiten 79-84, XP004103614 ISSN: 0167-2738**
- **MASHIMA K ET AL: "Ligand exchange reactions of a 1,3-butadiene complex of magnesium" JOURNAL OF ORGANOMETALLIC CHEMISTRY, ELSEVIER-SEQUOIA S.A. LAUSANNE, CH, Bd. 545-546, 30. Oktober 1997 (1997-10-30), Seiten 549-552, XP004103370 ISSN: 0022-328X**
- **DATABASE WPI Section Ch, Week 199512 Derwent Publications Ltd., London, GB; Class A17, AN 1995-085449 XP002191311 & JP 07 010931 A (TONEN CORP), 13. Januar 1995 (1995-01-13)**
- **DATABASE WPI Section Ch, Week 199425 Derwent Publications Ltd., London, GB; Class B05, AN 1994-206379 XP002191312 & JP 06 145107 A (KANEBUCHI KAGAKU KOGYO KK), 24. Mai 1994 (1994-05-24)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 209 141 B1

## Beschreibung

Magnesiumalkoholate (oder Magnesiumalkoxide) $Mg(OR)_2$, R = Alkyl, sind hydrolyseempfindliche Feststoffe, die in der organischen Synthese als selektive Basen und als Bestandteile von Polymerisationskatalysatoren eingesetzt werden können. Aufgrund der Fähigkeit des zweiwertigen Magnesium-Kations, stabile Chelatkomplexe mit Carbonylverbindungen zu bilden, wird beispielsweise Magnesiummethylat als selektives Carbonyl-Kondensationsmittel eingesetzt.

Magnesiummethylat wird durch Umsetzung von Magnesiummetall mit trockenem Methanol hergestellt (D. Caine, "Magnesium Methoxide" in: L. Paquette (ed), *"Encyclopedia of Reagents for Organic Synthesis",* 3204-3205). Die Magnesiumsalze der "höheren" primären Alkohole, d.h. mit ≥ 2-C-Atomen können unter Normalbedingungen nicht mehr direkt durch Umsetzung von Magnesiummetall mit den reinen Alkoholen gewonnen werden. Vielmehr werden Katalysatoren benötigt, um die Reaktion in Gang zu setzen (H. Lund u. J. Bjerrum, *Chem. Ber. (B),* 64, 210 **(1931);** US 2,287,088; DE 1,230,004 (Rheinpreußen **1967**)):

$$Mg + 2\,ROH \xrightarrow{Kat} Mg(OR)_2 + H_2\uparrow \qquad (1)$$

Kat = Hg oder $J_2$

Eine andere Möglichkeit zur Reaktionsbeschleunigung besteht darin, die Herstellung unter Druck bei dann möglichen höheren Temperaturen vorzunehmen. Beispielsweise können das Ethylat und das n-Propylat bei etwa 130 °C unter einem Druck von ca. 10 atü innerhalb weniger Stunden hergestellt werden (DE OS 2261386).

Wie in der Lteratur (H. Thoms, M. Epple, H. Viebrock, A. Reller, *J. Mater. Chem.* **1995,** 5 (4), 589-594) beschrieben und durch eigene Versuchen bestätigt, versagt die direkte Synthese ausgehend vom Mg-Metall und dem jeweiligen Alkohol (ROH) im Falle R = verzweigter Alkylrest (z.B. Propan-2-ol, tert-Butanol, Octan-2-ol).

Von Nachteil der Mg-Alkoholatsynthese gemäß (1) ist die Beschränkung auf R = Methyl, falls reiner Alkohol eingesetzt wird. Die Mg-Salze längerkettiger primärer Alkohole lassen sich zwar auch aus dem jeweiligen Alkohol und Mg-Metall her stellen, jedoch nur beim Einsatz von Katalysatoren. Der Katalysator stellt einen zusätzlichen Kostenfaktor dar und bewirkt eine Verunreinigung des Alkoholats Dem Einsatz von Quecksilber stehen ernsthafte ökologische und arbeitsschutzmäßige Bedenken entgegen. Für die Drucksynthese werden aufwendige Herstellapparaturen benötigt.

Es ist weiterhin bekannt, dass sterisch anspruchsvoll substituierte Magnesiumalkoholate aus Bis(organo) magnesium $R'_2Mg$ und Alkoholen hergestellt werden können (H. Thoms, M. Epple, A. Reller, *Solid State lonics* 101-103 **(1997),** 79-84):

$$(C_2H_5)_2Mg + 2\,ROH \xrightarrow[-2\,C_2H_6]{} Mg(OR)_2\downarrow \qquad (2)$$

R = gesättigtes Alkyl, z.B. Ethyl

Der Nachteil von Syntheseweg (2) besteht hauptsächlich darin, dass von relativ teuren Dialkylmagnesiumverbindungen, wie z.B. $Et_2Mg$, ausgegangen werden muss. Dialkylverbindungen mit R = gesättigtem Alkylrest werden nämlich gewöhnlich aus Grignardverbindungen RMgX und Organolithiumverbindungen gemäß

$$R{-}MgX + R{-}Li \longrightarrow R_2Mg + LiX\downarrow \qquad (3)$$

X=Cl, Br; I

oder durch Anlagerung von Olefinen an aktives Magnesiumhydrid gemäß

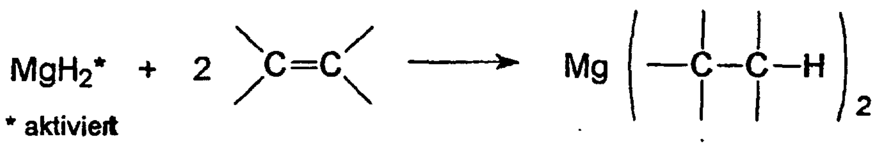


(4)

hergestellt. Bei Reaktion (3) werden die hohen Kosten durch den Preis der Organolithiumverbindung und der Grignardverbindung sowie durch die notwendige Entsorgung von Salzabfällen (LiX) verursacht. Außerdem ist das resultierende Mg-Alkoholat mit Lithiumresten aus der R-Li-Vorstufe verunreinigt, was der Reinheit (und damit der Selektivität bei Anwendungen als Katalysator) des $Mg(OR)_2$-Endproduktes abträglich ist. Bei Reaktion (4) ist von Nachteil, dass aktives Magnesiumhydrid kommerziell nicht erhältlich ist.

Aufgabe der Erfindung ist es, die Nachteile des Standes der Technik zu überwinden und insbesondere ein Verfahren zu schaffen, das ausgehend von allgemein verfügbaren Rohstoffen die Herstellung verschiedener Magnesiumalkoholate, auch die Mg-Alkoholate sekundärer und tertiärer Alkohole, zulässt.

Die Aufgabe wird dadurch gelöst, dass Additionsprodukte von 1,3-Dienen an Mg-Metall in einem polaren, aprotischen Lösungsmittel mit einem Alkohol R-OH (R = Alkylrest mit 2 bis 10 C-Atomen) umgesetzt werden. Das gebildete unlösliche Mg-Alkoholat $Mg(OR)_2$ kann anschließend durch eine fest/flüssig-Trennoperation isoliert werden.

Es wurde überraschend gefunden, dass die Additionsprodukte aus Mg-Metall und 1,3-Dienen reaktiv genug sind, um auch mit wenig aciden Alkoholen, wie z.B. tert-Butanol, schnell und quantitativ abzureagieren.

In der Literatur (K. Nützel, in Houben-Weyl, Methoden der Organischen Chemie, Band XIII/2a, **S**. 210-214, G. Thieme Verlag Stuttgart **1973)** ist das Additionsprodukt von Isopren und Magnesium beschrieben, das sich in Form eines 2 : 1 Adduktes bildet:

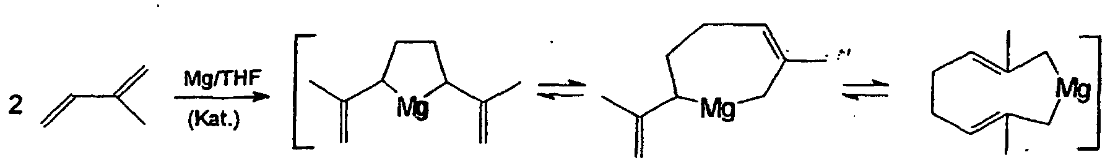


(5)

Überraschend wurde weiterhin gefunden, dass bei der Reaktion von 1,3-Dien mit Mg-Metall zum Additionsprodukt zur vollständigen Auflösung einer bestimmten Menge Magnesium weniger als die doppelte molare Menge an 1,3-Dien benötigt wird. Je nach Reaktionsbedingungen liegt im Additionsprodukt das molare Verhältnis 1,3-Dien zu Mg zwischen 2 : 1 und 1 : 1.

Die Umsetzung zwischen dem 1,3-Dien und Magnesium erfolgt im Temperaturbereich zwischen 0 und 100 °C, bevorzugt bei etwa 10 bis 70 °C. Das Mg-Metall wird bevorzugt mit großer Oberfläche, z.B. als Späne, Granulat oder Pulver eingesetzt.

Als 1,3-Dien finden bevorzugt 1,3-Butadien, Isopren, Dimethylbutadien und/oder 1,3-Cyclohexadien Verwendung.

Als Lösungsmittel dienen polare, aprotische Lösungsmittel, bevorzugt Ether, besonders bevorzugt THF, 2-Methyl-THF, Dimethylether oder Dimethoxyethan. Das polare, aprotische Lösungsmittel kann mit flüssigen Kohlenwasserstoffen, wie Pentan, Hexan, Cyclohexan, Heptan, Octan, Toluol oder Xylol, gemischt sein.

Bei Verwendung des besonders bevorzugten Lösungsmittels THF erfolgt die Umsetzung bevorzugt unter Rückflussbedingungen, wobei der Siedepunkt von der Art des 1,3-Diens abhängig ist. Wird 1,3-Butadien eingesetzt, empfiehlt es sich, die Umsetzung bei leicht erhöhtem Druck (bis 1,5 bar) auszuführen oder einen bei Temperaturen von < -10 °C arbeitenden Kühler zu verwenden, um keine nennenswerten Butadienverluste eintreten zu lassen. Beim Einsatz von Isopren kann bei Normaldruck und Temperaturen zwischen 50 und 65 °C gearbeitet werden.

Zur Reaktionsbeschleunigung kann ein als Metallphasentransferkatalysator wirkender Mehrkernaromat, wie z.B. Anthracen, Phenanthren oder Biphenyl, in katalytischen Mengen (bevorzugt 0,01 bis 5 Mol%, bezogen auf die Mg-Menge) zugesetzt werden. Als besonders vorteilhafter Katalysator hat sich Anthracen erwiesen. Der Katalysator wird besonders vorteilhaft (zur Vermeidung von Reaktionshemmungen) vor der Zudosierung des 1,3-Diens zur Suspension von Magnesiummetall im aprotischen Lösungsmittel gegeben. Das Magnesiumanthracenaddukt ist nämlich orange gefärbt und aus seiner optisch gut sichtbaren Entstehung kann der Operator leicht schließen, dass das Reaktionsgemisch inert ist (d.h. vollständig wasserfrei) und das Metall in aktivierter Form vorliegt. Dann bilden sich die Additionsprodukte des gewählten 1,3-Diens und des Magnesiums ungehemmt und schnell.

Das Molverhältnis zwischen dem eingesetzten 1,3-Dien und dem eingesetzten Mg kann bevorzugt zwischen

5 : 1 und 1 : 5 variieren. Besonders bevorzugt beträgt das 1,3-Dien : Mg-Verhältnis 1 : 1 bis 1 : 3. Wenn ein metallfreies Zwischen-, bzw. Endprodukt erhalten werden soll, muss in diesem Fall das lösliche 1,3-Dien/Mg-Additionsprodukt vom überschüssigen Mg-Metall abgetrennt werden.

Die erhaltene Lösung des 1,3-Dien/Magnesium-Additionsproduktes kann unter Luft- und Feuchtigkeitsausschluss mehrere Wochen bis Monate gelagert werden.

Zur Herstellung eines Mg-Alkoholates wird die Lösung des 1,3-Dien/Mg-Additionsproduktes mit dem gewünschten Alkohol versetzt. Als Alkohol kann z.B. iso-Propanol, tert-Butanol, 2-Ethylhexanol oder tert-Pentanol verwendet werden. Vorzugsweise wird das Additionsprodukt vorgelegt und der Alkohol in reiner Form oder mit einem Lösungsmittel verdünnt zugegeben. Bevorzugt wird der Alkohol in mindestens doppelter molarer Menge bezogen auf das 1,3-Dien/Mg-Additionsprodukt (also in mindestens stöchiometrischer Menge) zugegeben. Die Umsetzung ist stark exotherm. Die Reaktionstemperatur kann zwischen 0 und 100 °C, bevorzugt zwischen 20 bis 70 °C, liegen. Wird die Umsetzung in leicht flüchtigen Lösungsmitteln, wie z.B. THF, vorgenommen, kann unter Rückflussbedingungen gearbeitet werden. Die Zugabe des Alkohols erfolgt in der Weise, dass die entstehende Wärme sicher abgeführt werden kann, das ist je nach Maßstab und zur Verfügung stehenden Apparaten zwischen ca. 10 min und 5 Stunden. Das unlösliche Alkoholat wird durch Filtration oder Zentrifugation abgetrennt, gewaschen und anschließend getrocknet.

Die typische Ausbeute an Mg-alkoholat liegt bei > 95 % bei einer Reinheit zwischen 90 und 99 % (je nach Waschen). Das Produkt ist frei von Metallverunreinigungen, speziell liegt der Li-Gehalt < 100 ppm.

Die Herstellung des Mg-alkoholates kann auch ohne vorherige Isolierung des 1,3-Dien/Magnesium-Additionsproduktes in demselben Reaktionsgefäß erfolgen. Bei dieser Verfahrensvariante beträgt das bevorzugte Molverhältnis Gesamtmenge 1,3-Dien zu Mg 5 : 1 bis 1,5 : 1, besonders bevorzugt 3 : 1 bis 2 : 1, d.h. es wird nicht mit Mg-Überschuss gearbeitet.

Die Herstellung des Mg-alkoholates kann auch zumindest teilweise simultan zur Bildung weiteren 1,3-Dien/Magnesium-Additionsproduktes erfolgen. Dazu wird das Mg-Metall im aprotischen Lösungsmittel vorgelegt und zunächst mit 5 bis 99 % der Gesamtmenge an 1,3-Dien versetzt. Die Zugabe kann innerhalb von wenigen Minuten bis ca. 2 Stunden erfolgen. Auch hier kann ein Mehrkernaromat als Katalysator verwendet werden. Nach Beendigung der ersten Reaktionsphase wird der für die Alkoholatbildung benötigte Alkohol im Gemisch mit der restlichen Menge an 1,3-Dien bevorzugt innerhalb von 0,5 bis 5 Stunden zugegeben. Dabei ist darauf zu achten, dass das 1,3-Dien/Magnesium-Additionsprodukt im Hinblick auf die zudosierte Alkoholmenge stets im Überschuss vorliegt. Sollte dies nicht der Fall sein, kann die Umsetzung ganz zum Erliegen kommen. Die Kontrolle erfolgt entweder rein visuell (z.B. orangerote Färbung des Anthracen/Mg-Adduktes; diese Färbung verschwindet bei Alkohol Überschuss) oder durch spektroskopische Methoden (z.B. Fotometrie oder "Inline-Infrarot"). Dementsprechend kann die Zugabe des Alkohols auch automatisch gesteuert werden. Bevorzugt wird in der ersten Reaktionsphase 1 bis 50 % der Gesamtmenge des 1,3-Dien/Magnesium-Additionsproduktes gebildet, bevor die Umsetzung zu Mg-Alkoholat durch die Zugabe des Alkohols gestartet wird. In einer stark exothermen Reaktion bildet sich das Magnesiumalkoholat, das überraschenderweise zunächst in löslicher Form anfällt. Erst gegen Ende der Zudosierung beginnt das Alkoholat, als farbloser, feinteiliger Feststoff auszufallen, der in üblicher Weise isoliert wird.

Die so hergestellten Mg-Alkoholate können z.B. als Katalysator in der organischen Synthese eingesetzt werden, bevorzugt können sie als Kondensationsmittel verwendet werden.

Der Gegenstand der Erfindung wird anhand der folgenden Beispiele näher erläutert:

**Beispiel 1: Herstellung von Magnesium-tert-butoxid im "Eintopfverfahren"**

In einem 1-I-Doppelmantelglasreaktor mit Rückflusskühler, Innenthermometer und Tropftrichter wurden 9,0 g (370 mmol) Mg-Pulver (Timminco, < 150 µm) in 480 g wasserfreiem THF suspendiert und mit 1,9 g (11 mmol, 3 mol%) Anthracen versetzt. Dann wurde auf 50 °C erwärmt. Nach etwa 20 Minuten färbte sich die Lösung durch die Bildung des Mg-Anthracenadduktes orange. Daraufhin wurden 51 g (750 mmol) Isopren innerhalb von 10 min zugetropft und 1,5 Stunden bei einer Innentemperatur zwischen 50 und 55 °C gerührt. Es wurde eine Probe entnommen und auf Gesamtbasegehalt titriert: der Basengehalt von 0,29 mmol/g entsprach einem Umsatz von ca. 21 % des insgesamt eingesetzten Magnesiums. Dann wurde eine Mischung aus 58 g t-Butanol (780 mmol) und 6 g Isopren (90 mmol) innerhalb von 130 Minuten zugegeben. Dabei wurde darauf geachtet, dass die vom Mg-Anthracenkomplex herrührende Farbe nicht verschwand.

Durch die freiwerdende Reaktionswärme erhitzte sich das Gemisch bis zum Siedepunkt (je nach Reaktionsfortschritt 59 bis 65 °C). Nach Zugabe von etwa ¾ der Alkoholmenge begann ein farbloser Feststoff auszufallen.

Nach Ende der Zugabe wurde noch 30 Minuten refluxiert und die Reaktionsmischung auf eine Glasfilterfritte abgelassen. Das feste, farblose Produkt wurde auf der Fritte zweimal mit Hexan gewaschen und dann bei Raumtemperatur bis zur Gewichtskonstanz vakuumgetrocknet.

| | |
|---|---|
| Ausbeute | 58,6 g (344 mmol, 93 % der Theorie) |
| Analyse | Gesamtbase: 11,0 mmol/g (theoretisch 11,7) Mg: 5,51 mmo/g (theoretisch 5,86) |

Im Produkt waren keine Mg-Metallreste mehr zu erkennen.

**Beispiel 2: Herstellung des löslichen Isopren/Mg-Komplexes**

In einem 2-l-Doppelmantelreaktor mit Intensivrückflusskühler, Tropftrichter und Innenthermometer wurden 92 g Mg-Pulver (3,79 mol) in 1090 g THF suspendiert und mit 1,0 g (6 mmol, 0,2 mol %) Anthracen versetzt. Es wurde refluxiert, bis die orangerote Farbe des Anthracenkomplexes zu identifizieren war (ca. 30 Minuten) und dann auf 40 °C abgekühlt. Bei dieser Temperatur wurden innerhalb von 40 Minuten 207 g Isopren (3,04 mol) zugetropft. Nach Ende des Zutropfens wurde die Manteltemperatur auf 70 °C erhöht und 5 Stunden unter gutem Rühren refluxiert. Die Siedetemperatur stieg dabei von 59 auf 66 °C.

Dann wurde auf ca. 40 °C abgekühlt und die Suspension durch eine Glasfritte filtriert. Der Reaktor und die Fritte wurden mit 100 g THF ausgespült.

| | |
|---|---|
| Auswaage | 1419 g grünlichgelbe, leicht zähflüssige Lösung |
| Analyse | Gesamtbase: 2,91 mmol/g |

Die Ausbeute von 4129 mmol Base entspricht 2065 mmol löslichem Mg/Isopren-Komplex. Daraus ergibt sich, dass ca. 36 % 1:1-Komplex neben 64 % 2:1-Addukt vorliegen müssen.

**Beispiel 3: Herstellung von Magnesium-tert-butoxid aus isoliertem Mg/Isopren-Addukt**

In einem 2-l-Doppelmantelreaktor mit Tropftrichter, Rückflusskühler und Innenthermometer wurden 634 g der filtrierten Lösung aus Beispiel 2 (= 922 mmol Mg-Addukt) bei einer Innentemperatur von 30 °C vorgelegt. Bei konstanter Manteltemperatur von 30 °C wurde eine Mischung aus 143,8 g (1,94 mol, 5 % Überschuß) wasserfreies tert-Butanol im Gemisch mit 17 g THF innerhalb 15 Minuten zudosiert. Das Reaktionsgemisch erhitzte sich dabei schnell bis zum Siedepunkt (61 °C). Nach Dosierende wurde noch 80 Minuten refluxiert. Es bildete sich eine weiße Suspension, die nach Abkühlung auf 40 °C auf eine Glasfilterfritte abgelassen wurde. Nach Entfernung der Mutterlauge wurde der weiße feste Filterrückstand mit drei Portionen Hexan (je ca. 200 g) gewaschen.

Der Filterkuchen wurde dann zunächst bei Raumtemperatur, dann bei 60 bis 90 °C vakuumgetrocknet.

| | | |
|---|---|---|
| Ausbeute | 155 g feines, weißes Pulver (95 % der Theorie) | |
| Analyse | Gesamtbase | 11,34 mmol/g |
| | $Mg^{2+}$ | 5,64 mmol/g |

**Patentansprüche**

1. Verfahren zur Herstellung von Magnesiumalkoholaten $Mg(OR)_2$ mit R = Alkylrest mit 2 bis 10 C-Atomen, **dadurch gekennzeichnet, dass** Additionsprodukte von 1,3-Dienen an Mg-Metall in einem polaren, aprotischen Lösungsmittel mit einem Alkohol R-OH umgesetzt werden.

2. Verfahren nach Anspruch 1, **dadurch gekennzeichnet, dass** Mg-Metall mit einem 1,3-Dien zum 1,3-Dien/Magnesium-Additionsprodukt umgesetzt wird, dieses isoliert wird und zu einem späteren Zeitpunkt mit dem Alkohol zum Mg-Alkoholat umgesetzt wird.

3. Verfahren nach Anspruch 1, **dadurch gekennzeichnet, dass** das 1,3-Dien/Magnesium-Additionsprodukt in demselben Reaktionsgefäß, in dem die weitere Umsetzung zum Mg-Alkoholat erfolgt, aus Mg-Metall und einem 1,3-Dien hergestellt wird.

4. Verfahren nach Anspruch 3, **dadurch gekennzeichnet, dass** die Bildung des Mg-Alkoholates zumindest teilweise simultan zur Bildung weiteren 1,3-Dien/Magnesium-Additionsproduktes erfolgt.

5. Verfahren nach Anspruch 4, **dadurch gekennzeichnet, dass** nach Bildung von 1 bis 50 % der Gesamtmenge des 1,3-Dien/Magnesium-Additionsproduktes die weitere Umsetzung zu Mg-Alkoholat durch Zugabe des Alkohols gestartet wird.

6. Verfahren nach einem der Ansprüche 1 bis 5, **dadurch gekennzeichnet, dass** als 1,3-Dien 1,3-Butadien, Isopren, Dimethylbutadien und/oder 1,3-Cyclohexadien verwendet wird.

7. Verfahren nach einem der Ansprüche 1 bis 6, **dadurch gekennzeichnet, dass** als polares, aprotisches Lösungsmittel ein Ether verwendet wird.

8. Verfahren nach Anspruch 7, **dadurch gekennzeichnet, dass** als Ether Tetrahydrofuran, 2-Methyl-Tetrahydrofuran, Dimethylether oder 1,3-Dimethoxyethan oder eine Mischung daraus verwendet wird.

9. Verfahren nach einem der Ansprüche 1 bis 8, **dadurch gekennzeichnet, dass** das polare, aprotische Lösungsmittel zusätzlich einen flüssigen Kohlenwasserstoff enthält.

10. Verfahren nach einem der Ansprüche 1 bis 9, **dadurch gekennzeichnet, dass** die Bildung des Additionsproduktes durch einen Mehrkernaromaten katalysiert wird.

11. Verfahren nach Anspruch 10, **dadurch gekennzeichnet, dass** der Mehrkemaromat Anthracen, Phenanthren oder Biphenyl ist.

12. Verfahren nach Anspruch 10 oder 11, **dadurch gekennzeichnet, dass** der Mehrkernaromat ein einer Menge von 0,01 bis 5 Mol-% bezogen auf die Magnesiummenge zugegeben wird.

13. Verfahren nach einem der Ansprüche 1 bis 12, **dadurch gekennzeichnet, dass** als Alkohol iso-Propanol, tert-Butanol, 2-Ethylhexanol oder tert-Pentanol verwendet wird.

## Claims

1. Method of preparing magnesium alcoholates $Mg(OR)_2$ where R = alkyl residue having 2 to 10 carbon atoms, **characterised in that** addition products of 1,3-dienes to Mg metal in a polar, aprotic solvent are reacted with an alcohol R-OH.

2. Method according to claim 1, **characterised in that** Mg metal is reacted with a 1,3-diene to give the 1,3-diene/magnesium addition product, the latter is isolated and at a later point in time is reacted with the alcohol to give the Mg alcoholate.

3. Method according to claim 1, **characterised in that** the 1,3-diene/magnesium addition product is prepared from Mg metal and a 1,3-diene in the same reaction vessel in which the further reaction to form the Mg alcoholate takes place.

4. Method according to claim 3, **characterised in that** at least part of the formation of the Mg alcoholate takes place simultaneously with the formation of further 1,3-diene/magnesium addition product.

5. Method according to claim 4, **characterised in that**, after 1 to 50% of the total quantity of the 1,3-diene/magnesium addition product has formed, the further reaction to produce Mg alcoholate is started by adding the alcohol.

6. Method according to one of claims 1 to 5, **characterised in that** the 1,3-diene used is 1,3-butadiene, isoprene, dimethyl butadiene and/or 1,3-cyclohexadiene.

7. Method according to one of claims 1 to 6, **characterised in that** the polar, aprotic solvent used is an ether.

8. Method according to claim 7, **characterised in that** the ether used is tetrahydrofuran, 2-methyl tetrahydrofuran, dimethyl ether or 1,3-dimethoxyethane or a mixture thereof.

9. Method according to one of claims 1 to 8, **characterised in that** the polar, aprotic solvent also contains a liquid

hydrocarbon.

10. Method according to one of claims 1 to 9, **characterised in that** the formation of the addition product is catalysed by a polynuclear aromatic compound.

11. Method according to claim 10, **characterised in that** the polynuclear aromatic compound is anthracene, phenanthrene or biphenyl.

12. Method according to claim 10 or 11, **characterised in that** the polynuclear aromatic compound is added in a quantity of 0.01 to 5 mol% relative to the quantity of magnesium.

13. Method according to one of claims 1 to 12, **characterised in that** the alcohol used is isopropanol, tert. butanol, 2-ethyl hexanol or tert. pentanol.

## Revendications

1. Procédé de préparation d'alcoolates de magnésium, de formule $Mg(OR)_2$ dans laquelle R représente un groupe alkyle comportant de 2 à 10 atomes de carbone, **caractérisé en ce que** l'on fait réagir avec un alcool de formule R-OH des produits d'addition de 1,3-diènes sur du magnésium métallique, dans un solvant aprotique polaire.

2. Procédé conforme à la revendication 1, **caractérisé en ce que** l'on fait réagir du magnésium métallique sur un 1,3-diène, ce qui donne un produit d'addition de 1,3-diène sur du magnésium, produit que l'on isole et que l'on fait ultérieurement réagir avec l'alcool pour obtenir l'alcoolate de magnésium.

3. Procédé conforme à la revendication 1, **caractérisé en ce que** l'on prépare un produit d'addition de 1,3-diène sur du magnésium, à partir de magnésium métallique et d'un 1,3-diène, dans le même récipient réactionnel où l'on effectuera la réaction ultérieure donnant l'alcoolate de magnésium.

4. Procédé conforme à la revendication 3, **caractérisé en ce que** la formation de l'alcoolate de magnésium s'effectue, au moins en partie, en même temps que se poursuit la formation du produit d'addition de 1,3-diène sur du magnésium

5. Procédé conforme à la revendication 4, **caractérisé en ce que** c'est après qu'il s'est formé 1 à 50 % de la quantité totale de produit d'addition de 1,3-diène sur du magnésium que l'on fait démarrer la réaction ultérieure donnant l'alcoolate de magnésium, par addition de l'alcool.

6. Procédé conforme à l'une des revendications 1 à 5, **caractérisé en ce que** le 1,3-diène employé est du 1,3-butadiène, de l'isoprène, du diméthylbutadiène et/ou du 1,3-cyclohexadiène.

7. Procédé conforme à l'une des revendications 1 à 6, **caractérisé en ce que** le solvant aprotique polaire employé est un éther.

8. Procédé conforme à la revendication 7, **caractérisé en ce que** l'éther employé est du tétrahydrofurane, du 2-méthyl-tétrahydrofurane, de l'éther diméthylique ou du 1,2-diméthoxyéthane, ou encore un mélange de ces éthers.

9. Procédé conforme à l'une des revendications 1 à 8, **caractérisé en ce que** le solvant aprotique polaire contient en plus un hydrocarbure liquide.

10. Procédé conforme à l'une des revendications 1 à 9, **caractérisé en ce que** la formation du produit d'addtion est catalysée par un composé aromatique polycyclique.

11. Procédé conforme à la revendication 10, **caractérisé en ce que** le composé aromatique polycyclique est de l'anthracène, du phénanthrène ou du biphényle.

12. Procédé conforme à la revendication 10 ou 11, **caractérisé en ce que** l'on ajoute le composé aromatique polycyclique en une quantité de 0,01 à 5 % en moles, par rapport à la quantité de magnésium.

**13.** Procédé conforme à l'une des revendications 1 à 12, **caractérisé en ce que** l'alcool employé est de l'isopropanol, du tertiobutanol, du 2-éthylhexanol ou du tertiopentanol.